# EUROPEAN PATENT SPECIFICATION

(11) **EP 0 804 088 B1**
(45) Date of publication and mention of the grant of the patent: **25.10.2000**
(21) Application number: 94919820.4
(22) Date of filing: 29.06.1994
(51) Int. Cl.: A23K 1/17, A61K 9/14, A61K 47/36

(54) **STABILIZED COMPOSITION COMPRISING COLISTIN SULFATE**
COLISTINSULFAT ENTHALTENDE STABILISIERTE ZUSAMMENSETZUNG
COMPOSITION STABILISEE A BASE DE SULFATE DE COLISTINE

(43) Date of publication of application: 05.11.1997
(73) Proprietor: Meiji Seika Kaisha, Ltd., Tokyo 104-8002 (JP)
(72) Inventor: NAKATA, Toshiyuki, 788, Kayama Odawara-shi Kanagawa 250 (JP); TAKAOKA, Noboru, 788, Kayama Odawara-shi Kanagawa 250 (JP); IIZUKA, Masamoto, 788, Kayama Odawara-shi Kanagawa 250 (JP); OKABE, Yoshiaki, 788, Kayama Odawara-shi Kanagawa 250 (JP); KINOSHITA, Motoharu, Pharma-Technology Res.Lab., Odawara-shi, Kanagawa 250 (JP); KOMATSU, Yoshio, 4-16, Kyobashi 2-chome Chuo-ku Tokyo 104 (JP); NAGASATO, Toshiaki, 4-16, Kyobashi 2-chome Chuo-ku Tokyo 104 (JP)
(74) Representative: Popp, Eugen, Dr.
(86) International application number: JP9401054
(87) International publication number: WO9600506

(56) References cited:
- EP-A- 0 226 175
- EP-A- 0 387 597
- GB-A- 1 349 620
- GB-A- 1 485 210
- DATABASE WPI Week 9432, Derwent Publications Ltd., London, GB; AN 94-259506 & JP,A,6 189 691 (MEIJI SEIKA KAISHA) 12 July 1994
- DATABASE WPI Week 9331, Derwent Publications Ltd., London, GB; AN 93-247523 & JP,A,5 168 421 (KAKEN PHARM CO LTD) 2 July 1993
- DATABASE WPI Week 7950, Derwent Publications Ltd., London, GB; AN 79-90502B & JP,B,54 038 163 (KYOWA HAKKO KOGYO KK) 19 November 1979
- PATENT ABSTRACTS OF JAPAN vol. 17, no. 259 (C-1061) 21 May 1993 & JP,A,05 003 759 (ASAHI CHEM IND CO LTD) 14 January 1993
- PATENT ABSTRACTS OF JAPAN vol. 9, no. 45 (C-268) 26 February 1985 & JP,A,59 187 744 (UENO SEIYAKU OUYOU KENKYUSHO KK) 24 October 1984
- DATABASE WPI Week 8534, Derwent Publications Ltd., London, GB; AN 85-207789 & JP,A,60 132 987 (RIKEN VITAMIN CO KK) 16 July 1985

## Description

This invention relates to a new stabilized composition comprising colistin sulfate as active ingredient, which is essentially in the form of composite particles and is useful to be used as an additive to feedstuff for raising useful animals and to be used as a medicine for protection of useful animals from bacterial infections, and in which colistin sulfate presented as the active ingredient is incorporated in said composition in such a manner that colistin sulfate can be prevented from bringing about its partial degradation and partial reduction in its antibacterial activity or potency even when the said composition is mixed with a feedstuff and the resulting mixture is then shaped into the form of pellets by compression under heating to a high temperature in the presence of hot water vapor, or even when the said composition alone is stored for a long time at an elevated temperature of 40°C under air. This invention also relates to a process for the production of such new stabilized composition comprising colistin sulfate.

### BACKGROUND ART

Colistin is known cyclopeptide antibiotic which is usually composed of colistins A, B and C (see the "Merck Index", 11th edition, page 2476 (1989)). Colistin sulfate which is now commercially available is usually a mixture of colistin A sulfate and colistin B sulfate. The term "colistin sulfate" referred to hereinafter means colistin A sulfate itself or a commercially available mixture of colistin A sulfate with a smaller amount of colistin B sulfate.

Colistin sulfate has officially been announced as an antibiotic substance which is permitted to be used as an additive to the feedstuff for raising useful animals, for example, pig, cow and chicken, for a purpose of promoting the growth of useful animals as raised and also for purpose of enhancing the efficiency of digestion of the feedstuff as fed to useful animals. Further, colistin sulfate has widely been used as an antibacterial medicine for administration to useful animals for a purpose of protecting the animals from being attacked by diarrhoea or scours caused due to bacterial infections.

Hithertobefore, however, it is known that when a conventional blended feedstuff which has been shaped in the pellet form and is containing colistin sulfate incoporated therein as the feedstuff additive for the above-mentioned purposes is stored for a period of one month or longer at an elevated temperature of 40°C, the antibacterial activity or potency exhibited by the blended feedstuff so stored can be reduced with lapse of time to an unfavorably low level, indicating that the colistin sulfate component presented as the antibacterially active ingredient in the above-mentioned pellet type of the blended feedstuff is not stable within the blended feedstuff. On the other hand, it is also known that when colistin sulfate alone is stored at ambient temperatures for a long period of time under air, colistin sulfate is not inactivated to a substantial extent and does not reduce its antibacterial potency with the lapse of time. In these circumstances, it is not known before what are exact reasons why colistin sulfate is not stable when colistin sulfate is incorporated as the additive into the blended feedstuff of the pellet-shaped type which is then kept at 40°C for one month or longer.

Accordingly, there exists an outstanding demand for obtaining a new stabilized and antibacterial composition comprising colistin sulfate as active ingredient, in which colistin sulfate component can be stabilized against reduction of its antibacterial activity or potency to an unfavorably low level, and which can ensure that even if a blended feedstuff of the pellet-shaped type containing such a new stabilized and antibacterial composition as incorporated in said feedstuff is stored at 40°C for one month or a little longer, the blended feedstuff so stored does not exhibit a substantially reduced antibacterial activity or potency during or after the storage.

An object of this invention is to provide such a new stabilized and antibacterial composition having the properties as now demanded and comprising colistin sulfate as active ingredient. Another object of this invention is to provide a process for the production of such new stabilized and antibacterial composition comprising colistin sulfate, which is commercially workable in a facile and efficient way. Another objects of this invention will be clear from the following descriptions.

### DISCLOSURE OF INVENTION

In order to achieve the above-mentioned objects of this invention, we, the present inventors, have made extensive researches. As a result thereof, we have found that presumable exact reasons why the colistin sulfate component present in the blended feedstuff of the pellet-shaped type can reduce its antibacterial potency with laspe of time during the storage at 40°C and, in this sense, is not stable are that the colistin sulfate compoment present can gradually be degraded or structurally modified and thus can be inactivated partially or entirely during the storage of the blended feedstuff of the pellet-shape type as prepared, with involving any changes in the molecular structure of the colistin cyclopeptide, and that the degradation and inactivation of the colistin sulfate component take place because of the following causative matters.

Thus, these matters are firstly such that divalent metal ions, e.g., iron ion, magnesium ion, calcium ion or the like which are concomitantly existing as impurities or as the usual mineral additive in the blended feedstuff as employed will migrate and diffuse into the particles of the colistin sulfate component present, when a mixture of the blended feedstuff and colistin sulfate is shaped by compression into the form of pellets by heating under pressure at an elevated temperature of 120°C or more or less in the presence of hot water vapor; secondly that the di-valent metal ions as diffused into the particles of the colistin sulfate component will be brought into direct contact with and interact with the colistin sulfate component under the actions of heat and pressure in the presence of a water content supplied from the water vapor as employed; thirdly that the interaction of the di-valent metal ions with the colistin sulfate component will probably produce unknown chelated compounds which are not soluble in water or some organic solvents; and fourthly that the above-mentioned interaction of the di-valent metal ions with the colistin sulfate component will progress further during the storage of the pelleted feedstuff composition at an elevated temperature of 40°C or more or less.

Incidentally, we have found through our experiments that when colistin sulfate itself is brought into contact with the di-valent metal ions such as iron ion, magnesium ion or the like at ambient temperatures of up to 25°C and under atmospheric pressure, colistin sulfate normally does not reduce its antibacterial potency by a substantial extent with lapse of time.

Through our further investigations, we have now discovered that the above-mentioned degradation and inactivation of the colistin sulfate component which will occasionally take place in the blended feedstuff of the pellet-shaped type can be inhibited or eliminated by ensuring that the di-valent metal ions concomitantly existing in the feedstuff can be prevented from migrating into direct contact with the colistin sulfate component during the course of the process of preparing the blended feedstuff of pellet-shaped type, as well as during the time period of storing the pellet-shaped feedstuff so prepared. In order to prevent the divalent metal ions from migrating into direct contact with the colistin sulfate component in the above-mentioned way and thereby interacting with the colistin sulfate component to degrade and inactivate colistin sulfate under the above-mentioned circumstances, we now have found it necessary to provide the colistin sulfate component in the form of composite particles which are each formed from a mixture or combination of 1 part by weight of micro-particles of colistin sulfate, 0.1 to 3 parts by weight of α-starch of a gelatinized state and 0.5 to 5 parts by weight of micro-particles of β-starch and/or soybean meal, which have a low water content of not more than 12% by weight, of which the particle size falls in a range of from 250 µm to 750 µm, and which each have such an internal structure that the micro-particles of colistin sulfate and of β-starch and/or soybean meal are dispersed evenly within a continuous phase or matrix formed of the α-starch of the gelatinized state, so that almost all of the colistin sulfate micro-particles are embeded in the continuous phase or matrix made of α-starch and that the individual composite particle has the structure of a solid dispersion. Furthermore, we have found that if the composite partilces prepared as above and containing the colistin sulfate micro-particles dispersed therein are simply incorporated with omitting the micro-particles of β-starch and/or soybean meal as the concommitant constituent of the composite particles, or if the weight ratios between the colistin sulfate micro-particles, the continuous phase-forming α-starch material and the micro-particles of β-starch and/or soybean meal which are presented in the composite particles go beyond the predetermined range of 1 : 0.1 - 3.0 : 0.5 - 5.0, the degradation and inactivation of the colistin sulfate component can take place significantly during the course of the process of preparing the pellet-shaped feedstuff which even have contained such composite particles of colistin sulfate incorporated therein, as well as during the time of storing such pellet-shaped feedstuff as prepared.

Moreover, we have now found that the composite particles containing the disperse micro-particles of colistin sulfate and having the required particular composition and internal structure as described in the above may be produced in a facile and efficient way when there is employed our newly devised process which comprises mixing fine particles of colistin sulfate with a past-like aqueous solution of α-starch in water, agitating the resulting mixture to disperse the colistin sulfate particles well in the paste-like aqueous solution of α-starch and thereby to form a uniform paste, mixing the resulting uniform paste with micro-particles of β-starch and/or soybean meal, then kneading the resulting mixture of the colistin sulfate micro-particles, α-starch, water and the micro-particles of β-starch and/or soybean meal to give a kneaded, uniform and dough-like mass where the micro-particles of colistin sulfate as well as of β-starch and/or soybean meal are dispersed evenly within the hydrous, continuous phase or matrix made of the α-starch in said mass, dividing the resulting dough-like mass into smaller pieces with aid of a suitable mechanical means, drying these smaller pieces to an appropriate reduced water content, grinding the dried pieces to obtain a ground material comprising the separate composite particles of different sizes, and sieving the ground material to recover the separate composite particles having a desired particle size range.

According to a first aspect of this invention, therefore, there is provided a stabilized composition containing colistin sulfate as active ingredient and being in the form of a powder or granules, characterized in that said composition of the form of a powder comprises separate composite particles made of a mixture consisting of 1 part by weight of colistin sulfate, 0.1 to 3 parts by weight of α-starch and 0.5 to 5.0 parts by weight of β-starch or soybean meal or an admixture of β-starch and soybean meal and having a water content of not more than 12% by weight based on the weight of said mixture, that the separate composite particles made of said mixture have a particle size in a range of from 250 µm to 750 µm, and that each of the separate composite particles made of said mixture has such an internal structure that colistin sulfate and β-starch and/or soybean meal are present as their separate micro-particles which are dispersed evenly within a continuous phase or matrix made of the α-starch present in the individual composite particle of said mixture, so that almost all of the micro-particles of colistin sulfate and β-starch and/or soybean meal are embeded in the continuous phase or matrix made of the α-starch, and that, if desired, the stabilized composition may be in the form of granules as shaped by compressing the separate composite particles of said mixture into the granular form.

The new stabilized composition containing colistin sulfate according to the first aspect of this invention is stabilized in such sense that the antibacterial potency exhibited by the new composition of this invention is stable and thus is not reduced significantly even after the new composition of this invention is mixed with a blended feedstuff and then the resulting mixture is shaped into the pellet form by heating said mixture under pressure in the presence of hot water vapor at an elevated temperature of 120°C or more or less, and also when the pellet-shaped feedstuff prepared as above and containing the new composition of this invention mixed therein is packed in a sealed container and stored at 40°C for a time of one month or a little longer under air at a high relative humidity of 75%.

Of course, the new stabilized composition of this invention can maintain its initial antibacterial potency even at 40°C for a long time of more than one month when said composition is solely stored in a sealed container under such severed storage conditions without having been mixed with any feedstuff. Accordingly, the new composition of this invention itself is useful as a stabilized antibacterial formulation of colistin sulfate which is directly administered to animals for the purpose of preventing the animal from attack of bacterial infections.

### BEST MODE FOR CARRYING OUT THE INVENTION

Colistin sulfate which is usable to be incorporated into the new stabilized composition of this invention may vary from a crude colistin sulfate product of a 3% purity or more to a purified product of a 50% purity or more. Alpha-starch which is usable in the new stabilized composition of this invention may be of a variety and may be originated from various cereals such as wheat, rice, corn etc., and also from various potatoes such as white potato, sweet potato, talo-potatoes etc. This applies to the β-starch which is usuable in the new stabilized composition of this invention.

The starch, either in the alpha-form or in the beta-form, which may be incorporated as the necessary constituents in the new stabilized composition of this invention may preferably be such ones which have a low content of the ash. However, severe limitations are not posed to the nature of the starch as employed. Preferred examples of α-starch and β-starch usuable in the new composition of this invention are those of corn-starch. Soybean meal usuable in the new composition of this invention may be originated from various sorts of soybean.

The α-starch which is incorporated into the new composition of this invention may be prepared beforehand by mixing fine particles of raw starch, namely β-starch with water and heating the resulting aqueous mixture at a temperature of 95°C or thereabout, then dehydrating the resulting gelatinized starch paste to an appropriate water content convenient for use, and if desired, pulverising the so dried paste. The dry pulver of α-starch so obtained may be mixed with water to form a paste-like aqueous solution of α-starch, which may further be mixed with the micro-particles of the other constituents for forming the new composition of this invention.

In the new stabilized composition of this invention, the separate composite particles comprised in the composition may preferably be made of a mixture consisting of 1 part by weight of colistin sulfate, 0.2 to 2 parts by weight of α-starch and 1 to 2 parts by weight of β-starch or soybean meal or an admixture of β-starch and soybean meal and having a water content of not more than 12% by weight based on the weight of said mixture.

Thus, in the composite particles according to this invention, the weight ratios between colistin sulfate, α-starch and β-starch may preferably in a range of 1.0 : 0.2 - 2.0 : 1.0 - 2.0 and more preferably in a range of 1.0 : 0.2 - 1.0 : 1.0 - 2.0.

The composite particles comprised in the composition of this invention may preferably contain 10 to 50% by weight of colistin sulfate and may preferably have a water content of 3% to 10% by weight based on the weight of the mixture which constitutes said composite particles.

The composite particles comprised in the composition of this invention should not necessarily have one particular particle size in the range of from 250 µm to 750 µm but may have a range of particle sizes in the range of 250 - 750 µm. The composite particles may preferably have any particle size in a range of from 350 µm to 500 µm.

If desired, the new stabilized composition of this invention may further comprise additional constituent(s) such as carboxymethylcellulose, in addition to the colistin sulfate and starches, as long as these additional constituent(s) do neither liberate the divalent metal ions, nor give any unfavorable effect to the colistin sulfate component.

According to a second aspect of this invention, there is further provided a process for the production of stabilized composition containing colistin sulfate as active ingredient, which composition is in the form of a powder comprising such separate composite particles of a mixture of colistin sulfate as active ingredient, α-starch, and β-starch and/or soybean meal, where micro-particles of colistin sulfate and β-starch and/or soybean meal are dispersed evenly within a continuous phase or matrix made of α-starch present in the individual composite particle of said mixture, characterized in that the process comprises the following steps;-
(a) mixing 1 part by weight of fine particles of colistin sulfate with a paste-like aqueous solution of 0.1 to 3 parts by weight of α-starch in water and agitating the resulting mixture until the colistin sulfate particles are well dispersed and partially dissolved in the paste-like aqueous solution of α-starch and the resulting mixture forms a uniform paste,
(b) mixing the resultant uniform paste as made of the mixture of colistin sulfate, α-starch and water, with 0.5 to 5 parts by weight of micro-particles of β-starch and/or micro-particles of soybean meal,
(c) kneading the resulting mixture of colistin sulfate, α-starch, water and the β-starch and/or soybean meal as added, until the mixture so kneaded becomes a uniform and dough-like mass wherein micro-particles of colistin sulfate and micro-particles of β-starch and/or micro-particles of soybean meal are dispersed evenly within a hydrous, continuous phase or matrix made of the α-starch present in said mass,
(d) dividing the resulting uniform and dough-like mass into smaller pieces,
(e) drying the resultant small pieces to a water content of up to 10% by weight based on the weight of said small pieces,
(f) grinding the so dried small pieces to obtain a ground material comprising separate composite particles as formed and having different particle sizes, of which each composite particle is made of the mixture of colistin sulfate, α-starch and β-starch and/or soybean meal and has such an internal structure that micro-particles of colistin sulfate and micro-particles of β-starch and/or soybean meal are dispersed evenly within a continuous phase or matrix made of α-starch present in said composite particle, so that almost all of the micro-particles of colistin sulfate and β-starch and/or soybean meal are embeded in the continuous phase or matrix of the α-starch present in each of said composite particles of different particle sizes,
(g) sieving the above-mentioned ground material to recover a powder comprising the composite particles having a particle size in a range of from 250 µm to 750 µm, and
(h) if desired, shaping said powder obtained in the step (g), by compression to form granules.

In the process according to the second aspect of this invention, the steps (a) and (b) as well as the steps (c), (d), (f), (g) and (h) of the process may respectively be carried out in a known per se manner at room temperature using conventional devices suitable for the respective ends intended in each step. The drying step (e) of the process may be carried out at an elevated temperature of up to 100°C or more in an appropriate but conventional drying appratus under reduced pressure.

The stabilized composition of colistin sulfate according to this invention may be utilized as an additive to feedstuffs.

The new stabilized composition comprising colistin sulfate as active ingredient according to the first aspect of this invention may be mixed in an appropriate proportion into a conventional feedstuff for raising livestock animals such as pig, cattle and chicken. Such conventional feedstuff may be known feed materials such as flours of corn, wheat, barley, soybean, rice or other cereals, rice bran and wheat bran or may also be a conventional blended seedstuf of a dry powdery form, namely of a dry mash type which is composed of corn (maize) flour, wheat flour, defatted soybean meal, fish meal, skimmed milk, vitamins, minerals, e.g. iron sulfate, magnesium carbonate, amino acids and flavoring agents.

The new stabilized composition comprising colistin sulfate according to this invention may be mixed with a conventional blended feedstuff in such proportions suitable for giving a colistin sulfate content of 10 ppm to 100 ppm in the resulting mixture, and the resulting mixture may then be shaped into the pellet form by means of a conventional pelleting machine by compressing said mixture at an elevated temperature of 120°C or more or less in the presence of hot water vapor.

In the course of the method of shaping the above-mentioned mixture into the pellet form, the colistin sulfate component of the new stabilized composition of this invention as mixed into the feedstuff materials can be kept stabilized, that is to say, can be prevented from being inactivated by an unfavorably high extent. Besides, the colistin sulfate component present in the blended feedstuff as shaped into the pellets in association with the new stabilized composition of colistin sulfate according to this invention can be kept stabilized, that is to say, can be prevented from being inactivated by an unfavorably high extent, so long as said blended feedstuff as pelleted is placed and sealed in a moisture-impermeable container and then stored at an elevated temperature of 40°C or thereabout for a long time of one month or more or less.

This invention is now illustrated with reference to the following Examples.

### Example 1

### (1) Production of the new stabilized composition comprising colistin sulfate according to this invention

A respresentative example of the new stabilized composition of colistin sulfate according to this invention was produced by the procedures of the following stages (i) to (viii):-
In stage (i), pulverised raw corn starch (20 g) was mixed with water (100 g) and the mixture obtained was heated to 95°C under good agitation until the raw starch was gelatinized to the state of α-starch and a paste-like aqueous solution of α-starch in water was formed.
In stage (ii), the paste-like aqueous solution of α-starch in water (totally 120 g) as obtained in the above stage (i) was mixed with 100 g of a fine powder of a commercially available colistin sulfate (composed of a mixture of colistin A sulfate and colistin B sulfate and having a colistin sulfate content of 50% by weighty, and the resulting mixture was agitated well at room temperature until the colistin sulfate was dispersed and partially dissolved in the paste-like aqueous solution of α-starch and the whole mixture formed a uniform paste.
In stage (iii), the uniform paste (totally 220 g) as obtained from the above stage (ii) was mixed with 200 g of a fine powder of raw corn starch (as the β-starch referred to hereinbefore).
In stage (iv), the mixture (totally 420 g) as obtained from the above stage (iii) was well kneaded at room temperature until the mixture so kneaded became a uniform and dough-like mass where the micro-particles of colistin sulfate and the micro-particle of raw corn starch (as the β-starch) were dispersed evenly or uniformly within the paste-like, hydrous and continuous phase made of the α-starch present in said mass.
In stage (v), the dough-like mass as obtained from the above kneading stage (iv) was divided into smaller pieces of appropriate dimensions of approximately 5 mm x 5 mm x 10 mm by means of an extruder and a cutting machine.
In stage (vi), the small pieces obtained from the above stage (v) was dried at 80°C to a water content of 5% to 9% in a drying apparatus under reduced pressure, to obtain the dried pieces.
In stage (vii), the dried pieces obtained from the stage (vi) were ground at room temperature in a grinding apparatus to obtain a ground material which comprised separate composite particles as formed and having different particle sizes.

These composite partcles were each made of the mixture or combination of the micro-particles of colistin sulfate, the micro-particles of raw corn starch (as the β-starch) and the continuous phase of α-starch and had such internal structure or arrangement that the micro-particles of colistin sulfate and of the raw corn starch (as the β-starch) were dispersed evenly within the continuous phase made of α-starch present in each composite particle.

In stage (viii), the ground material comprising the composite particles as obtained from the stage (vii) were sieved by means of several sieves having appropriate mesh sizes. Then, the composite particles having particle sizes ranging from about 250 µm to 300 µm were recovered as a desired composition of a powder form.

This desired composition comprising the composite particles having particles sizes ranging from 250 µm to 300 µm is an exemplified formulation of the new stabilized composition comprising colistin sulfate according to the first aspect of this invention, and it will be referred to as test formulation No. 1 hereinafter.

### (2) Preparation of formulations to be tested

The concentration of colistin sulfate in the above test formulation No. 1 was measured and evaluated as about 20% by weight, and the test formulation No. 1 was mixed with an appropriate amount of defatted rice bran at a weight ratio of about 1 : 1 so that the concentration of colistin sulfate in the resulting mixture was diluted to 10% by weight. This mixture of the test formulation No. 1 with rice bran will be referred to as test formulation No. 2.

40 g-Portions of the test formulation No.2 as above was further mixed with 10 kg-portions a commercially available blended feedstuff which was usually called as "standard pig starter for experiment (dry mash type)" and which was composed of corn flour, wheat flour, wheat bran, defatted soybean meal, fish meal and skimmed milk as the principal constituents and also contained vitamins, iron compounds, magnesium compounds and other minerals, amino acids and flavoring agents supplemented as the additives. The resulting admixture of the test formulation No. 2 with said blended feedstuff so simply mixed together had a colistin sulfate concentration of 40 ppm and showed the form of powder type mash and will be referred to as test formulation No. 3 hereinafter.

This test formulation No. 3 of the mash form was then shaped into pellets by compression at 120°C with steaming under pressure by means of a commercially available pelleting machine, so that the blended feedstuff having the pellet-shape was obtained in the form of cyrindrical pellets of 3 mm in diameter by 2 - 4 mm in length.

This pellet-shaped feedstuff containing the test formulation No. 1 associated therewith will be referred to as test formulation No. 4 hereinafter.

For the comparison purpose, a conventional and commercially available composition of a granular form comprising colistin sulfate as active ingredient was prepared by mixing the commercially available colistin sulfate with defatted rice bran and granulating the mixture in a fluidized bed to give the granules containing 40% by weight of colistin sulfate. This conventional granular composition of colistin sulfate was simply mixed with an appropriate amount of rice bran so that the resulting mixture contained 10% by weight of colistin sulfate. This resulting mixture will be referred to as test formulation No. 5 (comparative) hereinafter.

40 g-Portions of the test formulation No. 5 (comparative) was further mixed with 10 kg-portions of the commercially available blended feedstuff which was the same as that called as the "standard pig starter for experiment (dry mash type)".

The resulting admixture so obtained had the mash form and a colistin sulfate concentration of 40 ppm and will be referred to as test formulation No. 6 (comparative) hereinafter.

This test formulation No. 6 (comparative) of the mash form was then shaped into the pellets by the same pelleting methods as described above. The feedstuff in the cyrindrical pellet form of 3 mm in diameter by 2-4 mm in length was produced and will be referred to as test formulation No. 7 (comparative) hereinafter.

### (3) Test procedures for estimation of stability of colistin sulfate component in the test formulations

The test formulations No. 3 of the mash form and the test formulation No. 4 of the pellet form, as well as the test formulation No. 6 (comparative) of the mash form and the test formulation No. 7 (comparative) of the pellet form were respectively placed in 100 g-portions thereof into moisture-impermeable bags made of polyethylene sheet, followed by heat-sealing the openings of the bags.

The sealed bags containing the test formulations therein were then separately placed in bags each made of three overlapped layers of kraft paper sheet, followed by sealing the openings of the kraft paper bags.

These kraft paper bags containing the test formulations as packed in the bags of polyethylene sheet were stored for 4 weeks at 40°C under air at a relative humidity of 75%. During this storage for a period of up to 4 weeks, it was observed that any moisture did not permeate into the body of the test formulations contained in the polyethylene bags. At the start of the storage and at the ends of one week, two weeks, three weeks and four weeks from the start of the storage, two samples were taken in 20 g-portions from each test formulation contained in each polyethylene bag.

The residual concentration of colistin sulfate remaining in each sample as taken was determined by extracting the sample with a mixed solvent of pyridine and n-hexane, thereby transferring the entire quantity of the active colistin sulfate from the sample into the organic extract and measuring quantatively the amount of the active colistin sulfate present in the organic extract by a high performance liquid chromatography (HPLC).

Percentages of the residual concentration so determined of colistin sulfate in the samples were calculated on the basis of the initial concentration of colistin sulfate (40 ppm) in the test formulations, No. 3 and No. 6. And, the percentages so calculated were termed as "Residual Percentage" of colistin sulfate and were recorded for the tested formulations with lapse of the time of storage.

The above tests were carried out in duplicates for each tested formulation. The four numerical data thus obtained for each tested formulation were averaged. The averaged values of the residual percentages of colistin sulfate were used to estimate the stability of the colistin sulfate component present in the tested formulations.

### (4) Results of the tests

The residual percentages of the active colistin sulfate component remaining in the tested formulations as stored were evaluated as above, and they are summarized in table 1a below.

**Table 1a**

| Tested speciemens | Residual percentages of colistin sulfate after storage, (%) | | | |
|---|---|---|---|---|
| | After 1 week | After 2 weeks | After 3 weeks | After 4 weeks |
| Test formulation No. 4 (of the pellet form according to this invention) | 83.8 | 81.4 | 74.1 | 78.2 |
| Test formulation No. 7 (comparative) (of the pellet form according to the prior art) | 69.8 | 64.4 | 52.8 | 50.2 |
| Test formulation No. 3 (of the mash form | 100 | 100 | 98.3 | 97.3 |
| Test formulation No. 6 (comparative) (of the mash form) | 100 | 100 | 100 | 96.7 |

With reference to Table 1a above, it is to be noticed that the test formulation No. 4 was such product as produced by shaping into the pellets the admixture of the blended feedstuff with the aforesaid test formulation No. 2 which comprised the composite particles containing the colistin sulfate component as stabilized according to the first aspect of this invention.

It is observed that the test formulation No. 4 during its storage for 4 weeks showed a small and allowable decrease in the residual % values of the colistin sulfate component which ranged from 83.8% at 1st week to 78.2% at 4th week of the storage, indicating that the colistin sulfate component which was present in the composite particles of the test formulation No. 1 of this invention as mixed into the test formulation No. 4 was stabilized to a degree, in spite that said colistin sulfate component had been subjected to various degrading conditions which occurred in the process of shaping the test formulation No. 3 into the pellet form of the test formulation No. 4 by compression at 120°C.

In contrast, the test formulation No. 7 (comparative) was such product which was produced by shaping into the pellets the test formulation No. 6 (comparative) containing the granulated colistin sulfate according to the prior art. It is to be observed that the test formulation No. 7 (comparative) during its storage for 4 weeks showed a great and unallowable decrease in the residual % values of the colistin sulfate component which ranged from 69.8% at 1st week to 50.2% at 4th week, indicating that the colistin sulfate component present in the test formulation No. 7 (comparative) of the pellet form was likely to be degraded and inactivated too much unfavorably during the process of shaping the test formulation No. 6 (comparative) of the mash form into the pellet form of the test formulation No. 7 (comparative) by compression at 120°C and also during the storage.

With the test formulation No. 3 and the test formulation No. 6 (comparative) which are both of the mash form, it is observed that the residual % values of colistin sulfate was only slightly decreased from the initial value of 100% to about 97% at 4th week. This is probably because the colistin sulfate component present in these test formulations No. 3 and No. 6 had not been degraded substantially during the process of preparing these test formulations of the mash form and also during the time period of storing these test formulations of the mash form under the above storage conditions.

Moreover, for a sake of a reference, the aforesaid test formulation No. 1 which was a representatively exemplified formulation of the new stabilized composition of colistin sulfate according to this invention was tested by the same test procedure and under the same storage conditions as described hereinbefore, in order to estimate the stability of the colistin sulfate component in said test formulation No. 1.

The residual percentage of the colistin sulfate component in the test formulation No. 1 during its storage was calculated similarly but on the basis of the initial concentration of colistin sulfate (about 20% by weight) in the test formulation No. 1.

The test results so obtained are summarized in Table 1b below.

**Table 1b**

| Test speciemens | Residual percentages of colistin sulfate after storage, (%) | | | | |
|---|---|---|---|---|---|
| | At zero week | After 1 week | After 2 weeks | After 3 weeks | After 4 weeks |
| Test formulation No. 1 (of the form of the composite particles) | 100 | 100 | 100 | 100 | 99 |

From Table 1b above, it will be clear that the colistin sulfate component present in the test formulation No. 1 was entirely stable and was substantially not degraded at 40°C for 4 weeks of the storage.

### Example 2

The procedures of Example 1 were repeated as a second series of the production of the test formulations and of the estimation of the stability of the test formulations as produced. The test results so obtained are summarized in Table 2 below.

**Table 2**

| Tested speciemens | Residual percentages of colistin suflate after storage, (%) | | | |
|---|---|---|---|---|
| | After 1 week | After 2 weeks | After 3 weeks | After 4 weeks |
| Test formulation No. 4 (of the pellet form according to this invention) | 93.2 | 88.9 | 84.1 | 77.5 |
| Test formulation No. 7 (comparative) (of the pellet form according to the prior art) | 72.7 | 70.9 | 60.0 | 54.5 |
| Test formulation No. 3 (of the mash form) | 100 | 100 | 100 | 97.3 |
| Test formulation No. 6 (comparative) (of the mash form) | 100 | 100 | 98.8 | 95.4 |

With regard to Table 2 above, it will be clear that the observations similar to those as obtained from Table 1a of Example 1 could be attained.

### Example 3

### (1) Production of the new stabilized composition comprising colistin sulfate according to this invention

A further example of the new stabilized composition of colistin sulfate according to this invention was produced similarly to Example 1 but by a slightly modified procedures of the following stages (i) to (viii):-
In stage (i), a commercially available dry powder (20 g) of α-starch was mixed with water (100 g), and the mixture was agitated until the α-starch powder was dissolved in water to give a paste-like aqueous solution of α-starch in water.
In stage (ii), the paste-like aqueous solution of α-starch in water (totally 120 g) as obtained in the above stage (i) was mixed with 100 g of a fine powder of a commercially available colistin sulfate (composed of a mixture of colistin A sulfate and colistin B sulfate and having a colistin sulfate content of 50% by weight), and the resulting mixture was agitated well at room temperature until the collistin sulfate was dispersed and partially dissolved in the paste-like aqueous solution of α-starch and the whole mixture formed a uniform paste.
In stage (iii), the uniform paste (totally 220 g) as obtained from the above stage (ii) was mixed with 200 g of a fine powder of raw corn starch (as the β-starch referred to hereinbefore).
In stage (iv), the mixture (totally 420 g) as obtained from the above stage (iii) was well kneaded at room temperature until the mixture so kneaded became a uniform and dough-like mass where the micro-particles of colistin sulfate and the micro-particle of raw corn starch (as the β-starch) were dispersed evenly or uniformly within the paste-like, hydrous and continuous phase made of the α-starch present in said mass.
In stage (v), the dough-like mass as obtained from the above kneading stage (iv) was divided into smaller pieces of appropriate dimensions of approximately 5 mm x 5 mm x 10 mm by means of an extruder and a cutting machine.
In stage (vi), the small pieces obtained from the above stage (v) was dried at 80°C to a water content of 5% to 9% in a drying apparatus under reduced pressure, to obtain the dried pieces.
In stage (vii), the dried pieces obtained from the stage (vi) were ground at room temperature in a grinding apparatus to obtain a ground material which comprised separate composite particles as formed and having different particle sizes.

These composite particles were each made of the mixture or combination of the micro-particles of colistin sulfate, the micro-particles of raw corn starch (as the β-starch) and the continuous phase of α-starch and had such internal structure or arrangement that the micro-particles of colistin sulfate and of the raw corn starch (as the β-starch) were dispersed evenly within the continous phase made of α-starch present in each composite particle.

In stage (viii), the ground material comprising the composite particles as obtained from the stage (vii) were sieved by means of several sieves having appropriate mesh sizes. Then, the composite particles having particle sizes ranging from about 250 µm to 300 µm were recovered as a desired composition of a powder form.

This desired composition comprising the composite particles having particles sizes ranging from 250 µm to 300 µm is a further exemplified formulation of the new stabilized composition comprising colistin sulfate according to the first aspect of this invention, and it will be referred to as test formulation No. 8 hereinafter.

It is to be noted that the above-mentioned stages (i) to (viii) of this Example 3 are respectively the same as the stages (i) to (viii) of Example 1 given hereinbefore, except that the commercially available dry powder of α-starch was used at first as the starting material in the stage (i) of this Example 3, while the pulverized raw starch, namely β-starch was used as the starting material and was gelatinized to α-starch by hydrating with water at 95°C in the stage (i) of Example 1.

### (2) Preparation of formulations to be tested.

The test formulation No. 8 as obtained in the stage (viii) of this Example 3 was used to prepare therefrom different formulations to be tested. The preparation of these different formulations to be tested here was conducted in the same manner as in Example 1, (2) described hereinbefore.

Thus, the concentration of colistin sulfate in the test formulation No. 8 was measured and evaluated as about 20% by weight, and the test formulation No. 8 was mixed with an appropriate amount of defatted rice bran at a weight ratio of about 1 : 1 so that the concentration of colistin sulfate in the resulting mixture was diluted to 10% by weight. This mixture of the test formulation No. 8 with rice bran will be referred to as test formulation No. 9.

40 g-Portions of the test formulation No. 9 as above was further mixed with 10 kg-portions of a commercially available blended feedstuff which was usually called as "standard pig starter for experiment (dry mash type)" and which was the same as that employed in Example 1, (2).

The resulting admixture so obtained by simply mixing the test formulation No. 9 with said blended feedstuff had again a colistin sulfate concentration of 40 ppm and showed the form of the powder type mash, and this will be referred to as test formulation No. 10.

This test formulation No. 10 of the mash form was then shaped into pellets by compression at 120°C with steaming under pressure by means of a commercially available pelleting machine, so that the blended feedstuff having the pellet-shape was obtained in the form of cyrindrical pellets of 3 mm in diameter by 2 - 4 mm in length. This pellet-shaped feedstuff containing the test formulation No. 8 associated therewith will be referred to as test formulation No. 11 hereinafter.

For the comparison purpose, the test formulation No. 6 (comparative) of the mash form, as well as the test formulation No. 7 (comparative) of the pellet form were prepared and provided, which were respectively the same as the test formulation No. 6 (comparative) and the test formulation No. 7 (comparative) as produced in the same manner as in Example 1, (2).

### (3) Test procedures for estimation of stability of colistin sulfate component in the test formurations

The stability of the colistin sulfate component present in the test formulations as prepared in the above were then estimated by the same test procedures as described in Example 1, (3).

Thus, the test formulation No. 10 of the mash form and the test formulation No. 11 of the pellet form, as well as the test formulation No. 6 (comparative) of the mash form and the test formulation No. 7 (comparative) of the pellet form were respectively placed in 100 g-portions thereof into the moisture-impermealble bags made of polyethylene sheet, followed by heat-sealing the openings of the polyethylene bags. These sealed bags were separately packed in the bags made of the kraft paper sheets which were the same as those employed in Example 1, (3).

The test formulations so packed were then stored at 40°C for 4 weeks under the same storage conditions as described in Example 1, (3).

Two samples were taken in 20 g-portions thereof at a time interval of one week from each of the test formulations as stored. The residual concentration of colistin sulfate remaining in each sample as taken was determined in the same manner as in Example 1, (3).

The "residual percentages" of colistin sulfate in the test formulations during their storage were evaluated and recorded in the same way as in Example 1, (3).

The test results so obtained are summarized in Table 3 below.

**Table 3**

| Tested speciemens | Residual percentages of colistin sulfate after storage, (%) | | | |
|---|---|---|---|---|
| | After 1 week | After 2 weeks | After 3 weeks | After 4 weeks |
| Test formulation No. 11 (of the pellet form according to this invention) | 94.4 | 80.3 | 75.9 | 76.9 |
| Test formulation No. 7 (comparative) (of the pellet form according to the prior art) | 73.7 | 52.4 | 53.8 | 48.5 |
| Test formulation No. 10 (of the mash form) | 99.2 | 100 | 100 | 98.3 |
| Test formulation No. 6 (comparative) (of the mash form) | 98.9 | 100 | 97.3 | 98.1 |

From the test results of Table 3 above, it will be observed that, for the test formulation No. 11 of the pellet form into which the composite particles of the test formulation No. 8 containing the colistin sulfate micro-particles therein were associated, the residual percentages of colistin sulfate decreased from 94.4% at 1st week to 76.9% at 4th week of the storage. This decrease by about 14% in the residual percentages of colistin sulfate during the storage of the test formulation No. 11 at 40°C for 4 weeks was allowable, indicating that the colistin sulfate component present in the test formulation No. 11 was stabilized to a meritable degree.

In contrast, for the test formulation No. 7 (comparative) of the pellet form according to the prior art, the residual percentages of colistin sulfate decreased from 73.7% at 1st week to 48.5% at 4th week of the storage. This decrease in the residual percentages of colistin sulfate to the value of 48.5% for 4 weeks of the storage indicates that the colistin sulfate component could be degraded and inactivated by about a half from the initial content of colistin sulfate to an unallowable extent.

### Example 4

This Example 4 illustrates how the stability of the colistin sulfate component in the new stabilized composition comprising colistin sulfate according to this invention is affected by changing the relative proportions between the colistin sulfate, α-starch and β-starch which are incorporated into the composite particles comprised in said new stabilized composition of this invention.

### (1) Production of the stabilized composition comprising colistin sulfate according to this invention

Further several examples of the new stabilized composition of colistin sulfate according to this invention were produced by carrying out the stages (i) to (viii) of Example 3, (1) similarly to Example 3, (1).

In this Example 4, however, the stages (i), (iii) and (viii) were so modified that varying amount (20 g, 50 g, 100 g or 200 g) of a commercially available dry powder of α-starch was dissolved in 100 g of water in the stage (i), that varying amount (200 g, 150 g, 100 g or zero g) of the pulverized raw corn starch (as the β-starch) was mixed in the stage (iii) with the mixture of the paste-like aqueous solution of α-starch with 100 g of the commercially available colistin sulfate (same as that employed in Example 1) and that four different powders each composed of the composite particles having particle sizes of from 300 µm to 500 µm were recovered from the ground material in the stage (viii).

Thus, from the sieving stage (viii), there were obtained further three examples of the new stabilized colistin sulfate composition of this invention which were such three test formulations No. 12, No. 13 and No. 14. From this stage (viii), there was also obtained "comparative" test formulations No. 15. These four test foumulations No. 12 to No. 15 were such products which respectively contained their constituents in the proportions as indicated in Table 4 below.

**Table 4**

| Products | Proportions of constituents | | | Weight ratio between Colistin sulfate: α-starch: β-starch |
|---|---|---|---|---|
| | Colistin sulfate micro-particles | α-starch | β-starch micro-particles | |
| Test formulation No. 12 | 100 g | 20 g | 200 g | 1 : 0.2 : 2.0 |
| Test formulation No. 13 | 100 g | 50 g | 150 g | 1 : 0.5 : 1.5 |
| Test formulation No. 14 | 100 g | 100 g | 100 g | 1 : 1 : 1 |
| Test formulation No. 15 (comparative) | 100 g | 200 g | zero | 1 : 2 : 0 |

### (2) Preparation of formulations to be tested

The test formulations No. 12 to No. 15 as obtained in the above-mentioned stage (viii) were respectively mixed with an appropriate amount of rice bran at a weight ratio of about 1 : 1 or thereabout so that the resulting four mixtures would contain 10% by weight of colistin sulfate.

These four mixtures as obtained just above will be referred to as test formulation No. 16, test formulation No. 17, test formulation No. 18 and "comparative" test formulation No. 19, respectively.

40 g-Portions of each of these test formulations No. 16 to No. 19 was further mixed with 10 kg-portions of the commercially available blended feedstuff, namely the aforesaid standard pig starter for experiment described in Example 1, (2), followed by shaping the resulting four admixtures of the mash form (colistin sulfate : 40 ppm) into pellets in the same manner as in Example 1, (2).

Thereby, there were produced test formulation No. 20, test formulation No. 21, test formulation No. 22 and "comparative" test formulation No. 23, respectively, which were all of the pellet form.

Consequently, the test formulation No. 20, No. 21 and No. 22 so produced were respectively such products which respectively contained the composite particles coming from the test formulation No. 12, No. 13 and No. 14 of Table 4, respectively. While, the "comparative" test formulation No. 23 so produced was such a product which contained the particles coming from the test formulation No. 15 (comparative) of Table 4.

### (3) Test procedures for estimation of stability of colistin sulfate component in the test formulations

The stability of the colistin sulfate component present in the test formulations No. 20 to No. 23 as prepared in the above were then estimated by the same test procedures as described in Example 1, (3).

Thus, the test formulation No. 20, the test formulation No. 21, the test formulation No. 22 and the test formulation No. 23 (comparative) which were all of the pellet form were respectively placed in 100 g-portions thereof into the moisture-impermeable bags made of polyethylene sheet, followed by heat-sealing the openings of the polyethylene bags. These sealed bags were separately packed in the bags made of the kraft paper sheets which were the same as those employed in Example 1, (3).

The test formulations so packed were then stored at 40°C for 2 weeks under the same storage conditions as described in Example 1, (3).

Two samples were taken in 20 g-portions thereof at a time interval of one week from each of the test formulations as stored for 2 weeks. The residual concentration of colistin sulfate remaining in each sample as taken was determined in the same manner as in Example 1, (3).

For the comparison purpose, the test formulation No. 7 of the pellet form mentioned in Example 1, (2) was again tested in the same manner as above.

The "residual percentages" of colistin sulfate in the test formulations during their storage were evaluated and recorded in the same way as in Example 1, (3).

The test results so obtained are summarized in table 5 below.

**Table 5**

| Test speciemens | Residual percentages of colistin sulfate after storage, (%) | |
|---|---|---|
| | After 1 week | After 2 weeks |
| Test formulation No. 20 | 89.3 | 84.8 |
| Test formulation No. 21 | 90.7 | 83.9 |
| Test formulation No. 22 | 86.7 | 80.3 |
| Test formulation No. 23 (comparative) | 79.4 | 65.4 |
| Test formulation No. 7 (comparative) | 67.9 | 53.8 |

From the test results of Table 5 above, it will be observed that the residual percentage of colistin sulfate could be decreased to an unallowable level of 65.4% already after 2 weeks of the storage at 40°C with the test formulation No. 23 (comparative) which was produced using the test formulation No. 15 (comparative) of Table 4 and which was such product where the β-starch micro-particles were omitted from being incorporated in the formulation of colistin sulfate as employed.

It is also observed that, with the test formulations No. 20, No. 21 and No. 22, the colistin sulfate component incorporated therein was stabilized successfully to a practicably useful extent. This will indicate that the stabilized composition of colistin sulfate according to this invention is preferably such that the weight ratios between the colistin sulfate, the α-starch and the β-starch are in the range of 1.0 : 0.2 - 1.0 : 1.0 - 2.0, as demonstrated by the test formulations No. 12, No. 13 and No. 14 of Table 4 above.

### Example 5

This Example illustrates how the stability of the colistin sulfate component present in the new stabilized composition of colistin sulfate according to this invention is affected by the differences in the distribution of the particle size of the composite particles comprised in said stabilized composition of colistin sulfate.

### (1) Production of the new stabilized composition comprising colistin sulfate according to this invention

Further several examples of the stabilized composition of colistin sulfate according to this invention were produced by repeating the stages (i) to (viii) of Example 1, (1) above, but except that the sieving stage (viii) was effected so that there were recovered the seven different compositions of the powder form which were each composed of the composite particles having the different distributions of the particles size as indicated in Table 6a below.

These seven compositions as recovered will be referred to as test formulation No. 24, No. 25, No. 26, No. 27, No. 28, No. 29 and No. 30, respectively.

**Table 6a**

| Compositions of powder form as recovered | Range of particle size of composite particles present in the powder (µm) | Sieved powder fractions |
|---|---|---|
| Test formulation No.24 | 300 to 500 | passed 30-mesh sieve but retained on 50-mesh sieve |
| Test formulation No. 25 (comparative) | 150 to less than 250 | passed 50-mesh sieve but retained on 100-mesh sieve |
| Test formulation No. 26 | 500 to 710 | passed 22-mesh sieve but retained on 30-mesh sieve |
| Test formulation No. 27 | 355 to 500 | passed 30-mesh sieve but retained on 42-mesh sieve |
| Test formulation No. 28 | 300 to 355 | passed 42-mesh sieve but retained on 50-mesh sieve |
| Test formulation No. 29 | 250 to 300 | passed 50-mesh sieve but retained on 60-mesh sieve |
| Test formulation No. 30 | 250 to 710 | passed 22-mesh sieve but retained on 60-mesh sieve |

For the comparison purpose, there was taken the test formulation No. 5 (comparative) which was obtained in Example 1, (2) and which was produced by diluting the fluidized bed-granulated mixture of the colistin sulfate powder and rice bran (40% colistin sulfate content) with a further amount of rice bran to the concentration of colistin sulfate of 10% by weight.

This test formulation No. 5 (comparative) was ground and sieved so that a powder fraction having particles sizes of from 150 µm to 500 µm, as well as a powder fraction having particle size of from 250 µm to 710 µm. These two powder fractions will be referred to as test formulation No. 31 (comparative) and test formulation No. 32 (comparative), respectively. These test formulations No. 31 and No. 32 had the different distributions of particle size as indicated in Table 6b below.

**Table 6b**

| Compositions of Powder form | Range of particle size of the partides present in the powder (µm) | Sieved powder fraction |
|---|---|---|
| Test formulation No. 31 (comparative) | 150 to 500 | passed 30-mesh sieve but retained on 100-mesh sieve |
| Test formulation No. 32 (comparative) | 250 to 710 | passed 22-mesh sieve but retained on 60-mesh sieve |

### (2) Preparation of formulations to be tested

The test formulations No. 24 to No. 32 produced as above were used respectively to prepare different formulations to be tested for their stability of the colistin sulfate present in them. The preparation of these different formulations to be tested was conducted in the sane manner as in Example 1, (2).

Thus, each of the test formuations No. 24 to No. 32 was mixed with an appropriate amount of rice bran at a weight ratio of about 1 : 1 or more or less so that the concentration of colistin sulfate in each of the resulting nine different mixtures was diluted to 10% by weight. 40 g-portions of the nine different mixtures so resulted were further mixed with 10 kg-portions of the blended feedstuff same as the standard pig starter for experiment (dry mash type) employed in Example 1, (2).

In this way, there were prepared nine different feedstuff compositions of the mash form containing 40 ppm of colistin sulfate. The resulting nine compositions of the mash form were respectively shaped into the pellets in the same manner as in Example 1, (2), to give the nine different feedstuff compositions shaped in the pellet form.

### (3) Test procedures for estimation of the stability of the colistin sulfate component

The stability of the colistin sulfate component present in the nine different feedstuff compositions of the pellet form as prepared in the above was estimated in the same manner as in Example 1, (3) by measuring the residual percentages of colistin sulfate in the pellet-shaped feedstuff composition packed and stored at 40°C for 2 weeks in the same manner as detailed in Example 1, (3).

The test results so obtained are tabulated in Table 7 below.

**Table 7**

| Tested feedstuff compositions of the pellet form containing the colistin formulations indicated below | Residual percentages of colistin sulfate after storage, (%) | |
|---|---|---|
| | After 1 week | After 2 weeks |
| Test formulation No. 24 | 98.8 | 84.4 |
| Test formulation No. 25 (comparative) | 79.9 | 59.5 |
| Test formulation No. 26 | 92.8 | 80.5 |
| Test formulation No. 27 | 93.5 | 83.9 |
| Test formulation No. 28 | 93.5 | 81.2 |
| Test formulation No. 29 | 94.4 | 83.4 |
| Test formulation No. 30 | 93.9 | 83.1 |
| Test formulation No. 31 (comparative) | 72.4 | 48.5 |
| Test formulation No. 32 (comparative) | 75.7 | 65.4 |

From the test results of Table 7 above, it is observed that the residual percentage of colistin sulfate could be decreased to an unallowably low level of 79.9% already at 1st week and then to 59.5% at 2nd week of the storage with the pellet-shaped feedstuff composition containing the test formulation No. 25 (comparative) which, in turn, comprised the composite particles of particle sizes in the range of 150 to less than 250 µm not according to this invention.

### Example 6

This Example illustrate that the β-starch component micro-particles can be entirely or partially replaced by micro-particles of soybean meal as the necessary constituents of the composite particles according to this invention, and that an amount of carboxymethylcellulose (CMC) can additionally be incorporated into the composite particles according to this invention.

Further several examples of the new stabilized composition of colistin sulfate according to this invention were produced similarly to Example 3 by conducting the stages (i) to (viii) of Example 3 in the same manner as in Example 3, (1) but except that the uniform paste composed of a mixture of colistin sulfate (100 g), α-starch (20 g) and water (100 g) as obtained in the stage (ii) was mixed with fine powder of raw corn starch (as the β-starch) and/or fine powder of soybean meal in such different amounts indicated in Table 8, together with or without such amounts of carboxymethylcellulose (CMC) as indicated in Table 8 below.

Six different mixtures so obtained in the stage (iii) were respectively kneaded well in the subsequent stage (iv) to give six different uniform and dough-like masses as shown in Table 8 below.

**Table 8**

| Dough-like mass | Proportions of constituents in the dough | | | | | | Weight ratio between colistin sulfate: α-starch: β-starch: soybean meal |
|---|---|---|---|---|---|---|---|
| | Colistin sulfate g) | α-starch (g) | β-starch (g) | Soy-bean meal (g) | CMC (g) | Water (g) | |
| Dough A | 100 | 20 | 160 | - | 20 | 100 | 1 : 0.2 : 1.6 : 0 |
| Dough B | 100 | 20 | 80 | 80 | 20 | 100 | 1 : 0.2 : 0.8 : 0.8 |
| Dough C | 100 | 20 | 90 | 90 | - | 100 | 1 : 0.2 : 0.9 : 0.9 |
| Dough D | 100 | 20 | - | 180 | - | 100 | 1 : 0.2 : 0 : 1.8 |
| Dough E | 100 | 20 | 180 | - | - | 100 | 1 : 0.2 : 1.8 : 0 |
| Dough F | 100 | 20 | 180 | - | - | 100 | 1 : 0.2 : 1.8 : 0 |

The six different dough-like masses A, B, C, D, E and F as obtained in the stage (iv) and shown in Table 8 were respectively processed by the stages (v), (vi), (vii) and (viii). Thereby, six different compositions of the powder form were obtained, which each comprises the composite particles having particle sizes of about 250 µm to 300 µm and containing the constituents shown in Table 8 with a water content of 5-9% by weight.

These six different compositions comprising the composite particles will be referred to as test formulation No. 33, No. 34, No. 35, No. 36, No. 37 and No. 38, respectively.

### (2) Preparation of formulations to be tested

The test formulations No. 33 to No. 38 produced as above were used respectively to prepare different formulations to be tested for their stability of the colistin sulfate present in them. The preparation of these different formulations to be tested was conducted in the sane manner as in Example 1, (2).

Thus, each of the test formulations No. 33 to No. 38 was mixed with an appropriate amount of rice bran at a weight ratio of about 1 : 1 or more or less so that the concentration of colistin sulfate in each of the resulting six different mixtures was diluted to 10% by weight. 40 g-portions of the six different mixtures so resulted were further mixed with 10 kg-portions of the blended feedstuff same as the standard pig starter for experiment (dry mash type) employed in Example 1, (2).

In this way, there were prepared six different feedstuff compositions of the mash form containing 40 ppm of colistin sulfate. The resulting six compositions of the mash form were respectively shaped into the pellets in the same manner as in Example 1, (2), to give the six different feedstuff compositions shaped in the pellet form.

### (3) Test procedures for estimation of the stability of the colistin sulfate component

The stability of the colistin sulfate component present in the six different feedstuff compositions of the pellet form as prepared in the above was estimated in the same manner as in Example 1, (3) by measuring the residual percentages of colistin sulfate in the pelleted feedstuff composition packed and stored at 40°C for 2 weeks in the same manner as detailed in Example 1, (3).

The test results so obtained are tabulated in Table 9 below.

**Table 9**

| Tested feedstuff compositions of the pellet form containing the colistin formulations indicated below | Residual percentages of colistin sulfate after storage, (%) | |
|---|---|---|
| | After 1 week | After 2 weeks |
| Test formulation No. 33 | 85.2 | 71.2 |
| Test formulation No. 34 | 92.8 | 75.1 |
| Test formulation No. 35 | 95.3 | 77.0 |
| Test formulation No. 36 | 92.8 | 79.5 |
| Test formulation No. 37 | 91.9 | 73.0 |
| Test formulation No. 38 | 89.8 | 82.8 |

From the test results of Table 9 above, it is observed that the residual percentage of colistin sulfate was retained at an allowable level of 79.5%, namely about 80% at 2nd week of the storage with the pellet-shaped feedstuff composition containing the test formulation No. 36 (prepared using the dough D of Table 8), of which the composite particles of colistin sulfate contained the micro-particles of soybean meal in the absence of the micro-particles of β-starch.

This will indicate that the colistin sulfate component can be stabilized to a useful extent even when the β-starch micro-particles are entirely replaced by the micro-particles of soybean meal as the constituents of the composite particles of colistin sulfate according to this invention.

Besides, it was observed that the doughs B, C and D of Table 8 which contained the soybean meal and which was obtained in the above stage (iv) of Example 6, (1) were of higher viscosity or consistency than the doughs A, E and F which contained the β-starch in place of the soybean meal. The small pieces made of the doughs B, C or D could be dehydrated in the stage (vi) to a water content of 5-9% by weight much fastly than the small pieces of the doughs A, E or F.

### INDUSTRIAL APPLICABILITY

With the new stabilized composition comprising colistin sulfate as active ingredient according to this invention, the colistin sulfate component of this new stabilized composition is stabilized from being degraded and inactivated to an unallowably low level.

The stabilized composition of colistin sulfate according to this invention is useful to be mixed with a blended feedstuff of the mash form which is to be shaped into the pellet form by compression under heating. The pellet-shaped feedstuff composition so obtained can maintain its antibacetrial activity at an allowable level even after a long-time storage.

## Claims

1. A stabilized composition containing colistin sulfate as active ingredient and being in the form of a powder or granules, characterized in that said composition of the form of a powder comprises separate composite particles made of a mixture consisting of 1 part by weight of colistin sulfate, 0.1 to 3 parts by weight of a-starch and 0.5 to 5.0 parts by weight of β-starch or soybean meal or an admixture of β-starch and soybean meal and having a water content of not more than 12% by weight based on the weight of said mixture, that the separate composite particles made of said mixture have a particle size in a range of from 250 µm to 750 µm, and that each of the separate composite particles made of said mixture has such an internal structure that colistin sulfate and β-starch and/or soybean meal are present as their separate micro-particles which are dispersed evenly within a continuous phase or matrix made of the α-starch present in the individual composite particle of said mixture, so that almost all of the micro-particles of colistin sulfate and β-starch and/or soybean meal are embeded in the continuous phase or matrix made of the α-starch, and that, if desired, the stabilized composition may be in the form of granules as shaped by compressing the separate composite particles of said mixture into the granular form.

2. A stabilized composition as claimed in Claim 1, in which the separate composite particles comprised in the composition are made of a mixture consisting of 1 part by weight of colistin sulfate, 0.2 to 2 parts by weight of α-starch and 1 to 2 parts by weight of β-starch or soybean meal or an admixture of β-starch and soybean meal and having a water content of not more than 12% by weight based on the weight of said mixture.

3. Stabilized composition as claimed in Claim 1 or Claim 2, in which the composite particles comprised in the composition are made of a mixture of colistin sulfate, α-starch and β-starch where the weight ratios between colistin sulfate, α-starch and β-starch are in a range of 1.0 : 0.2 - 2.0 : 1.0 - 2.0, and preferably in a range of 1.0 : 0.2 - 1.0 : 1.0 - 2.0.

4. A stabilized composition as claimed in Claim 1, in which colistin sulfate is colistin A sulfate or is composed of a mixture of colistin A sulfate and colistin B sulfate.

5. A stabilized composition as claimed in Claim 1, in which each of the composite particles comprised in the composition contains 10 to 50% by weight of colistin sulfate.

6. A stabilized composition as claimed in Claim 1, in which the composite particles comprised in the composition have a water content of 3% to 10% by weight based on the weight of the mixture which constitutes said composite particles.

7. A stabilized composition as claimed in Claim 1, in which the composite particles comprised in the composition have a particle size in a range of from 350 µm to 500 µm.

8. A process for the production of stabilized composition containing colistin sulfate as active ingredient, which composition is in the form of a powder comprising such separate composite particles of a mixture of colistin sulfate as active ingredient, α-starch, and β-starch and/or soybean meal, where micro-particles of colistin sulfate and β-starch and/or soybean meal are dispersed evenly within a continuous phase or matrix made of α-starch present in the individual composite particle of said mixture, characterized in that the process comprises the following steps:-
(a) mixing 1 part by weight of fine particles of colistin sulfate with a paste-like aqueous solution of 0.1 to 3 parts by weight of α-starch in water and agitating the resulting mixture until the colistin sulfate particles are well dispersed and partially dissolved in the paste-like aqueous solution of α-starch and the resulting mixture forms a uniform paste,
(b) mixing the resultant uniform paste as made of the mixture of colistin sulfate, α-starch and water, with 0.5 to 5 parts by weight of micro-particles of β-starch and/or micro-particles of soybean meal,
(c) kneading the resulting mixture of colistin sulfate, α-starch, water and the β-starch and/or soybean meal as added, until the mixture so kneaded becomes a uniform and dough-like mass wherein micro-particles of colistin sulfate and micro-particles of β-starch and/or micro-particles of soybean meal are dispersed evenly within a hydrous, continuous phase or matrix made of the α-starch present in said mass,
(d) dividing the resulting uniform and dough-like mass into smaller pieces,
(e) drying the resultant small pieces to a water content of up to 10% by weight based on the weight of said small pieces,
(f) grinding the so dried small pieces to obtain a ground material comprising separate composite particles as formed and having different particle sizes, of which each composite particle is made of the mixture of colistin sulfate, α-starch and β-starch and/or soybean meal and has such an internal structure that micro-particles of colistin sulfate and micro-particles of β-starch and/or soybean meal are dispersed evenly within a continuous phase or matrix made of α-starch present in said composite particle, so that almost all of the micro-particles of colistin sulfate and β-starch and/or soybean meal are embeded in the continuous phase or matrix of the α-starch present in each of said composite particles of different particle sizes,
(g) sieving the above-mentioned ground material to recover a powder comprising the composite particles having a particle size in a range of from 250 µm to 750 µm, and
(h) if desired, shaping said powder obtained in the step (g), by compression to form granules.

## Patentansprüche

1. Stabilisierte Zusammensetzung, die Colistinsulfat als aktiven Bestandteil enthält und in Form eines Pulvers oder von Granulaten vorliegt, dadurch gekennzeichnet, daß die Zusammensetzung in Form eines Pulvers getrennte Verbundteilchen umfaßt, die aus einer Mischung aus 1 Gewichtsteil Colistinsulfat, 0,1 bis 3 Gewichtsteilen α-Stärke und 0,5 bis 5,0 Gewichtsteile β-Stärke oder Sojamehl oder einer Mischung aus β-Stärke und Sojamehl bestehen, und einen Wassergehalt von nicht mehr als 12 Gew.-%, bezogen auf das Gewicht der Mischung, besitzen, die getrennten Verbundteilchen aus dieser Mischung eine Teilchengröße im Bereich von 250 µm bis 750 µm besitzen, und jedes der getrennten Verbundteilchen aus der Mischung eine solche innere Struktur aufweist, daß Colistinsulfat und β-Stärke und/oder Sojamehl als getrennte Mikroteilchen vorhanden sind, die gleichmäßig innerhalb einer kontinuierlichen Phase oder Matrix aus der in den individuellen Verbundteilchen der Mischung vorhandenen α-Stärke dispergiert sind, wodurch fast alle der Mikroteilchen des Colistinsulfats und der β-Stärke und/oder des Sojamehls in der kontinuierlichen Phase oder Matrix aus der α-Stärke eingebettet sind, und dadurch, daß, wenn gewünscht, die stabilisierte Zusammensetzung in Form von Granulaten vorliegt, die durch Komprimieren der getrennten Verbundteilchen dieser Mischung in eine Granulatform ausgebildet sind.

2. Stabilisierte Zusammensetzung nach Anspruch 1, dadurch gekennzeichnet, daß die in der Zusammensetzung vorhandenen getrennten Verbundteilchen aus einer Mischung bestehen, die besteht aus 1 Gewichtsteil Colistinsulfat, 0,2 bis 2 Gewichtsteilen α-Stärke und 1 bis 2 Gewichtsteilen β-Stärke oder Sojamehl, oder einer Mischung aus β-Stärke und Sojamehl, und mit einem Wassergehalt von nicht mehr als 12 Gew.-%, bezogen auf das Gewicht der Mischung.

3. Stabilisierte Zusammensetzung nach Anspruch 1 oder 2, dadurch gekennzeichnet, daß die in der Zusammensetzung vorhandenen Verbundteilchen aus einer Mischung aus Colistinsulfat, α-Stärke und β-Stärke bestehen, wobei das Gewichtsverhältnis zwischen Colistinsulfat, α-Stärke und β-Stärke im Bereich von 1,0 : 0,2 - 2,0 : 1,0 - 2,0, und vorzugsweise im Bereich von 1,0 : 0,2 - 1,0 : 1,0 - 2,0 liegt.

4. Stabilisierte Zusammensetzung nach Anspruch 1, dadurch gekennzeichnet, daß das Colistinsulfat Colistin A-sulfat ist, oder aus einer Mischung aus Colistin A-sulfat und Colistin B-sulfat besteht.

5. Stabilisierte Zusammensetzung nach Anspruch 1, dadurch gekennzeichnet, daß jedes der in der Zusammensetzung enthaltenen Verbundteilchen 10 bis 50 Gew.-% Colistinsulfat enthält.

6. Stabilisierte Zusammensetzung nach Anspruch 1, dadurch gekennzeichnet, daß die in der Zusammensetzung vorhandenen Verbundteilchen einen Wassergehalt von 3 bis 10 Gew.-%, bezogen auf das Gewicht der Mischung, die die Verbundteilchen bildet, besitzen.

7. Stabilisierte Zusammensetzung nach Anspruch 1, dadurch gekennzeichnet, daß die in der Zusammensetzung vorhandenen Verbundteilchen eine Teilchengröße im Bereich von 350 µm bis 500 µm besitzen.

8. Verfahren zur Herstellung einer stabilisierten Zusammensetzung, die Colistinsulfat als aktiven Bestandteil enthält, wobei die Zusammensetzung in Form eines Pulvers vorliegt, das getrennte Verbundteilchen einer Mischung aus Colistinsulfat als aktiven Bestandteil, α-Stärke, und β-Stärke und/oder Sojamehl umfaßt, wobei die Mikroteilchen des Colistinsulfat und der β-Stärke und/oder des Sojamehls gleichmäßig in einer kontinuierlichen Phase oder Matrix aus α-Stärke verteilt sind, die in den individuellen Verbundteilchen der Mischung vorhanden ist, dadurch gekennzeichnet, daß das Verfahren die folgenden Stufen umfaßt:
(a) Mischen von 1 Gewichtsteil der feinen Teilchen von Colistinsulfat mit einer pastenförmigen wässerigen Lösung von 0,1 bis 3 Gewichtsteilen α-Stärke in Wasser und Schütteln der resultierenden Mischung, bis die Colistinsulfatteilchen gut dispergiert und teilweise in der pastenförmigen wässerigen Lösung der α-Stärke gelöst sind, und die resultierende Mischung eine gleichmäßige Paste bildet,
(b) Mischen der resultierenden aus der Mischung von Colistinsulfat, α-Stärke und Wasser hergestellten resultierenden gleichmäßigen Paste mit 0,5 bis 5 Gewichtsteilen von Mikroteilchen von β-Stärke und/oder Mikroteilchen von Sojamehl;
(c) Kneten der resultierenden Mischung aus Colistinsulfat, α-Stärke, Wasser und der zugegebene β-Stärke und/oder dem zugegebenen Sojamehl, bis die so geknetete Mischung eine gleichmäßige und teigartige Masse wird, worin die Mikroteilchen von Colistinsulfat und die Mikroteilchen von β-Stärke und/oder Mikroteilchen von Sojamehl gleichmäßig innerhalb einer wässerigen kontinuierlichen Phase oder Matrix aus der in der Masse vorhandenen α-Stärke dispergiert sind,
(d) Teilen der resultierenden gleichmäßigen und teigförmigen Masse in kleinere Anteile,
(e) Trocknen der resultierenden kleineren Anteile auf einen Wassergehalt von bis zu 10 Gew.-%, bezogen auf das Gewicht der kleineren Anteile,
(f) Mahlen der so getrockneten kleineren Anteile, um ein gemahlenes Material zu erhalten, das so geformte getrennte Verbundteilchen mit verschiedenen Teilchengrößen enthält, von denen jedes Verbundteilchen aus der Mischung aus Cholistinsulfat α-Stärke und β-Stärke und/oder Sojamehl besteht und eine solche innere Struktur besitzt, daß die Mikroteilchen aus Cholistinsulfat und die Mikroteilchen aus β-Stärke und/oder Sojamehl gleichmäßig innerhalb einer kontinuierlichen Phase oder Matrix aus in dem Verbundteilchen vorhandener α-Stärke dispergiert sind, wodurch fast alle Mikroteilchen aus Cholistinsulfat und β-Stärke und/oder Sojamehl in der kontinuierlichen Phase oder Matrix der in jedem dieser Verbundteilchen verschiedener Größe vorhandenen α-Stärke eingebettet sind,
(g) Sieben des vorstehend genannten gemahlenen Materials um ein Pulver zu erhalten, das die Verbundteilchen mit einer Teilchengröße im Bereich von 250 µm bis 750 µm enthält, und
(h) wenn gewünscht, Komprimieren des in Stufe (g) erhaltenen Pulvers in die Form von Granulaten.

## Revendications

1. Composition stabilisée contenant du sulfate de colistine en tant qu'ingrédient actif et étant sous la forme d'une poudre ou de granules, caractérisée en ce que ladite composition sous la forme d'une poudre comprend des particules composites séparées formées d'un mélange constitué de 1 partie en poids de sulfate de colistine, 0,1 à 3 parties en poids d'amidon a et 0,5 à 5,0 parties en poids d'amidon β ou de farine de soja ou d'un mélange d'amidon β et de farine de soja et ayant une teneur en eau de pas plus de 12 % en poids par rapport au poids dudit mélange, en ce que les particules composites séparées formées dudit mélange ont une dimension de particules comprise entre 250 µm et 750 µm, et en ce que chacune des particules composites séparées formées dudit mélange a une structure interne telle que le sulfate de colistine et l'amidon β et/ou la farine de soja sont présents sous la forme de leurs micro-particules séparées qui sont dispersées de façon uniforme dans une phase continue ou matrice formée de l'amidon α présent dans la particule composite individuelle dudit mélange, de sorte que presque toutes les microparticules de sulfate de colistine et d'amidon β et/ou de farine de soja sont incorporées dans la phase continue ou matrice formée de l'amidon α, et en ce que, si on le désire, la composition stabilisée peut être sous la forme de granules façonnés par compression des particules composites séparées dudit mélange en la forme granulaire.

2. Composition stabilisée selon la revendication 1, dans laquelle les particules composites séparées comprises dans la composition sont formées d'un mélange constitué de 1 partie en poids de sulfate de colistine, 0,2 à 2 parties en poids d'amidon α et 1 à 2 parties en poids d'amidon β ou de farine de soja ou d'un mélange d'amidon β et de farine de soja et ayant une teneur en eau de pas plus de 12 % en poids par rapport au poids dudit mélange.

3. Composition stabilisée selon la revendication 1 ou la revendication 2, dans laquelle les particules composites comprises dans la composition sont formées d'un mélange de sulfate de colistine, d'amidon α et d'amidon β, dans lequel les rapports en poids entre le sulfate de colistine, l'amidon α et l'amidon β sont dans une plage de 1,0 : 0,2 - 2,0 : 1,0 - 2,0, et de préférence dans une plage de 1,0 : 0,2 - 1,0 : 1,0 - 2,0.

4. Composition stabilisée selon la revendication 1, dans laquelle le sulfate de colistine est du sulfate de colistine A ou est composé d'un mélange de sulfate de colistine A et de sulfate de colistine B.

5. Composition stabilisée selon la revendication 1, dans laquelle chacune des particules composites comprises dans la composition contient de 10 à 50 % en poids de sulfate de colistine.

6. Composition stabilisée selon la revendication 1, dans laquelle les particules composites comprises dans la composition ont une teneur en eau de 3 % à 10 % en poids par rapport au poids du mélange qui constitue lesdites particules composites.

7. Composition stabilisée selon la revendication 1, dans laquelle les particules composites comprises dans la composition ont une dimension de particules dans une plage de 350 µm à 500 µm.

8. Procédé de production d'une composition stabilisée contenant du sulfate de colistine en tant qu'ingrédient actif, ladite composition se présentant sous la forme d'une poudre contenant de telles particules composites séparées formées d'un mélange de sulfate de colistine en tant qu'ingrédient actif, d'amidon α et d'amidon β et/ou de farine de soja, dans lequel des microparticules de sulfate de colistine et d'amidon β et/ou de farine de soja sont dispersées de façon uniforme dans une phase continue ou matrice formée d'amidon α présent dans la particule composite individuelle dudit mélange, caractérisé en ce que le procédé comprend les étapes suivantes :
(a) mixtion de 1 partie en poids de particules fines de sulfate de colistine avec une solution aqueuse, ressemblant à une pâte, de 0,1 à 3 parties en poids d'amidon α dans de l'eau et agitation du mélange résultant jusqu'à ce que les particules de sulfate de colistine soient bien dispersées et partiellement dissoutes dans la solution aqueuse d'amidon α ressemblant à une pâte et jusqu'à ce que le mélange résultant forme une pâte uniforme,
(b) mixtion de la pâte uniforme résultante, formée du mélange de sulfate de colistine, d'amidon α et d'eau, avec 0,5 à 5 parties en poids de microparticules d'amidon β et/ou de microparticules de farine de soja,
(c) malaxage du mélange résultant de sulfate de colistine, d'amidon α, d'eau et de l'amidon β et/ou de la farine de soja, tels qu'ajoutés, jusqu'à ce que ce le mélange ainsi malaxé devienne une masse uniforme et ressemblant à une pâte, dans lequel des microparticules d'amidon β et/ou des microparticules de farine de soja sont dispersées de façon uniforme dans une phase continue hydratée ou matrice formée de l'amidon a présent dans ladite masse,
(d) séparation de la masse uniforme et ressemblant à une pâte en morceaux plus petits,
(e) séchage des petits morceaux résultants jusqu'à une teneur en eau allant jusqu'à 10 % en poids par rapport au poids desdits petits morceaux,
(f) broyage des petits morceaux ainsi séchés pour obtenir une matière broyée comprenant des particules composites séparées ainsi formées et ayant différentes dimensions de particules, dont chaque particule composite est formée du mélange de sulfate de colistine, d'amidon α et d'amidon β et/ou de farine de soja et a une structure interne telle que les microparticules de sulfate de colistine et les microparticules d'amidon β et/ou de farine de soja sont dispersées de façon uniforme dans une phase continue ou matrice formée d'amidon α présent dans ladite particule composite, de sorte que presque toutes les microparticules de sulfate de colistine et d'amidon β et/ou de farine de soja sont incorporées dans la phase continue ou matrice de l'amidon α présent dans chacune desdites particules composites de différentes dimensions de particules,
(g) tamisage de la matière broyée susmentionnée pour recueillir une poudre comprenant les particules composites ayant une dimension de particules dans une plage de 250 µm à 750 µm, et
(h) si on le désire, façonnage de ladite poudre obtenue dans l'étape (g), par compression, pour former des granules.
